# DEMANDE DE BREVET EUROPEEN

(11) **EP 3 593 826 A2**
(43) Date de publication de la demande: **15.01.2020**
(21) Numéro de dépôt: 19183839.0
(22) Date de dépôt: 02.07.2019
(51) Int. Cl.: A61L 9/12, A44C 15/00

(54) **DISPOSITIF DE DIFFUSION D'UN PARFUM DANS UN BIJOU PORTATIF**

(30) Priorité: 13.07.2018 FR 1856461
(71) Demandeur: Albéa Services, 92230 Gennevilliers (FR)
(72) Inventeur: EZZINA, Emir, 78180 MONTIGNY-LE-BRETONNEUX (FR); MOURARET, Guillaume, 75017 PARIS (FR)
(74) Mandataire: Gevers & Orès

(57) **Abrégé**

Dispositif de diffusion d'un produit liquide, du type parfum, stocké dans un réservoir 3, comprenant :
- au moins une partie fixe 2 apte à être montée sur le réservoir 3, et offrant au moins un point d'alimentation en produit par capillarité, ladite partie fixe 2 comprenant un corps poreux fixe 9 apte à s'imbiber de produit par capillarité en étant directement plongé dans le réservoir 3 ;
- un élément nomade 1, pour chaque point d'alimentation, comprenant un corps poreux mobile 5 apte à s'imbiber de produit par capillarité en étant en contact avec le corps poreux fixe 9 lorsque l'élément nomade 1 est monté sur la partie fixe 2, ledit élément nomade 1 comprenant également une structure décorative 4, du type bijou 4, ledit corps poreux mobile 5 étant logé à l'intérieur de la structure décorative 4.

## Description

### Domaine de l'invention

L'invention concerne un dispositif de diffusion d'un produit liquide, stocké dans un réservoir. L'invention concerne également un flacon comprenant un réservoir dans lequel est stocké le produit, et comprenant un tel dispositif de diffusion du produit. Ce dispositif de diffusion de produit comporte un élément nomade que l'utilisateur peut emmener avec lui pour avoir une diffusion du produit tout au long de la journée.

Dans le cadre de l'invention, le produit liquide est un produit cosmétique, plus particulièrement un parfum, ou une eau de toilette.

### Etat de la technique

On connait des diffuseurs de senteurs pour la maison. Ces diffuseurs n'ont pas vocation à être déplacés, ils sont fixes.

On connaît également des applicateurs de parfum pour la peau, consistant en des éléments poreux destinés à être imbibé de parfum et à être ensuite appliqué sur la peau. Or certaines personnes ne souhaitent pas appliquer de parfum directement sur leur peau ou leurs vêtements, par exemple pour des raisons d'allergie, ou parce que certains parfums peuvent tâcher les vêtements.

Dans ce cas, il existe des bijoux diffuseurs de parfum, que l'utilisateur peut porter sur lui au courant de la journée. Ils consistent par exemple en un pendentif encapsulant un bout de tissu que l'utilisateur trempe au préalable dans le parfum avant de refermer le pendentif. Cela permet d'éviter le contact entre le parfum et la peau de l'utilisateur, ainsi que ses vêtements.

L'inconvénient réside dans la succession d'étapes à réaliser, en l'espèce ouvrir le pendentif, vaporiser du parfum sur le bout de tissu, positionner le tissu dans le pendentif, refermer le pendentif. Tout cela provoque la lassitude de l'utilisateur, et non appropriation du produit. De plus, le parfum présent sur le tissu entre en contact avec le pendentif et provoque une réaction chimique affectant le matériau du pendentif, comme la corrosion, etc.

### Résumé de l'invention

La présente invention a pour objectif de pallier les différents inconvénients énoncés ci-dessus, au moyen d'un dispositif de diffusion permettant de diffuser du parfum tout au long de la journée via un élément nomade emporté par l'utilisateur, qui soit facilement rechargeable en parfum avec un minimum d'étapes, et qui ne risque pas d'être altéré par réaction chimique avec le parfum.

Ce but est atteint grâce à un dispositif de diffusion d'un produit liquide, du type parfum, stocké dans un réservoir, comprenant :
- au moins une partie fixe apte à être montée sur le réservoir, et offrant au moins un point d'alimentation en produit par capillarité, ladite partie fixe comprenant un corps poreux fixe apte à s'imbiber de produit par capillarité en étant directement plongé dans le réservoir ;
- un élément nomade, pour chaque point d'alimentation, comprenant un corps poreux mobile apte à s'imbiber de produit par capillarité en étant en contact avec le corps poreux fixe lorsque l'élément nomade est monté sur la partie fixe, ledit élément nomade comprenant également une structure décorative, du type bijou, ledit corps poreux mobile étant logé à l'intérieur de la structure décorative, l'élément nomade comprenant des moyens de mise en retrait du corps poreux mobile par rapport à la structure décorative pour éviter tout contact direct entre eux.

L'idée principale de cette invention consiste à prévoir deux corps poreux, l'un fixe plongé dans le réservoir pour le prélèvement de parfum, et l'autre mobile intégré au sein d'un élément nomade que l'utilisateur peut emporter. La mise en contact entre ces deux corps poreux permet de transférer, par capillarité, le parfum depuis le réservoir vers l'élément nomade. Il n'est donc pas nécessaire de démonter l'élément nomade pour avoir accès à un élément poreux qu'il faut ensuite tremper dans le réservoir. Dans la présente invention, aucun élément n'est à démonter. Il suffit de déposer l'élément nomade sur son point d'alimentation, de manière à mettre en contact les deux corps poreux et que le transfert de parfum s'opère.

De plus, le corps poreux est logé à l'intérieur de la structure décorative, ce qui signifie que l'utilisateur ne touche pas le corps poreux mobile lorsqu'il manipule l'élément nomade. Il n'y a donc pas de contact entre la peau de l'utilisateur et le corps poreux mobile.

Enfin, le corps poreux mobile n'est pas en contact avec la structure décorative. En effet, il est en retrait, afin que le parfum imbibé dans le corps poreux mobile ne vienne pas toucher la structure décorative, et ne l'altère pas.

Le dispositif de diffusion peut comporter une seule partie fixe montée sur le réservoir.

Alternativement, le dispositif de diffusion peut comporter plusieurs parties fixes montées sur le même réservoir. Par exemple, le réservoir peut présenter plusieurs cols, chaque col recevant une partie fixe.

Chaque partie fixe peut présenter un unique point d'alimentation pour recharger un élément nomade en produit.

Alternativement, chaque partie fixe peut présenter plusieurs points d'alimentation pour recharger plusieurs éléments nomades en produit.

Selon les différents modes de réalisation de l'invention, qui pourront être pris ensemble ou séparément :
- la partie fixe comprend une base de fixation sur le réservoir retenant le corps poreux fixe en état de compression, ladite base étant ajourée par des fentes à travers lesquelles saillent des excroissances décomprimées du corps poreux fixe. Les portions du corps poreux fixe situées en face des fentes ont tendance à s'engouffrer dans les fentes, et donc à se décomprimer en formant des excroissances, ou des bossages.
- ladite base comporte un manchon fermé en partie supérieure par un chapeau et ouvert en partie inférieure de manière à définir un logement pour le corps poreux fixe, lesdites fentes étant pratiquées sur la paroi périphérique du manchon. Le corps poreux fixe est inséré dans le manchon via l'ouverture inférieure, et est maintenue en position au sein du manchon, par compression.
- le corps poreux fixe présente avantageusement une tête de diffusion, logée en partie supérieure du manchon, et une tige d'alimentation qui plonge au sein du réservoir, afin d'entrer en contact avec le produit, et de le ramener jusqu'à la tête par capillarité.
- ladite base comporte une jupe se développant autour de la partie inférieure du manchon, ladite jupe comprenant des moyens de fixation au col du réservoir. Par exemple, la jupe est encliquetée dans le col du réservoir. Elle peut également présenter un filetage intérieur pour être vissée au col du réservoir. D'autres systèmes de fixation peuvent être envisagés.
- la partie fixe comporte des moyens de protection contre l'évaporation du produit à travers lesdites fentes. En effet, le produit contenu dans le corps poreux fixe se diffuse à travers les fentes. Ainsi, le réservoir peut se vider par diffusion alors même qu'il n'y a pas d'élément nomade positionné sur la partie fixe. Il est ainsi primordial s'assurer une étanchéité autour du corps poreux fixe, qui est imbibé en toutes circonstances, lorsque l'élément nomade n'est pas mis en position sur la partie fixe.
- lesdits moyens de protection contre l'évaporation du produit consistent en une frette mobile en translation à l'encontre de moyens ressort entre une position haute de protection en vis-à-vis des fentes et une position basse de libre accès aux fentes pour l'élément nomade. Lorsque la frette est en position haute, elle entoure le manchon et obstrue toutes les fentes. L'étanchéité du corps poreux fixe est assurée.
- lesdits moyens ressort de ladite frette consistent en des pattes flexibles reposant au fond d'une gorge formée entre le manchon et la jupe de la base. La frette peut passer de la position haute à la position basse par flexion/déformation des pattes.
- le chapeau comporte un rebord périphérique contre lequel la frette vient en butée en position haute pour assurer l'étanchéité entre la base et la frette lorsque l'élément nomade n'est pas en place sur la partie fixe. Les pattes flexibles ont tendance à faire remonter la frette en position haute lorsqu'elles ne sont plus sollicitées. La frette remonte alors jusqu'à entrer en contact avec le rebord périphérique du chapeau. Ainsi, la frette se retrouve en butée sous le rebord, et bloque toute entrée/sortie d'air par rapport au manchon, de manière à empêcher la diffusion du parfum déjà imbibé sur le corps poreux fixe.
- le corps poreux mobile consiste en élément annulaire entourant la paroi périphérique ajourée du manchon lorsque l'élément nomade est en place sur la partie fixe, l'élément nomade exerçant une force sur la frette de manière à la maintenir en position basse par un effet de frottement. Lorsque l'utilisateur enfonce l'élément nomade sur la partie fixe, l'élément nomade pousse sur la frette et la fait passer de la position haute à la position basse, à l'encontre des moyens ressort. L'élément nomade est ensuite maintenu en position sur la partie fixe, grâce aux frottements axial et radial entre lui et le manchon. En effet, le diamètre intérieur de l'élément nomade est dimensionné par rapport au diamètre extérieur du manchon, de manière à ce que l'enfoncement de l'élément nomade sur le manchon se fasse avec suffisamment de frottements pour qu'il reste en position sur la partie fixe à court terme et à long terme si nécessaire. Les pattes flexibles ne sont pas suffisamment puissantes pour faire remonter la frette lorsque l'élément nomade est en place. A titre d'alternative, l'élément nomade peut être maintenu en position sur la partie fixe par encliquetage.
- lesdits moyens de mise en retrait du corps poreux mobile par rapport à la structure décorative de l'élément nomade consistent en une couronne supportant le corps poreux mobile, ladite couronne étant fixée de manière amovible dans la structure décorative.
- le corps poreux mobile est disposé à l'intérieur de la couronne, ledit corps poreux mobile venant directement au contact des excroissances décomprimées du corps poreux fixe lorsque l'élément nomade est en place sur la partie fixe.
- le corps poreux mobile est disposé à l'extérieur de la couronne, la couronne étant ajourée par des ouvertures à travers lesquelles des portions du corps poreux mobile sont accessibles, lesdites excroissances décomprimées du corps poreux fixe venant au contact de ces portions du corps poreux mobile lorsque l'élément nomade est en place sur la partie fixe.
- la couronne comporte une lèvre d'accroche supérieure et une lèvre d'accroche inférieure pour sa fixation avec la structure décorative, lesdites lèvres ayant une extension radiale dimensionnée de manière à assurer une mise en retrait du corps poreux mobile par rapport à la structure décorative.

Ainsi, il n'y a pas de contact entre le corps poreux mobile et la structure décorative, pour éviter toute détérioration de la structure décorative.
- le manchon comporte des languettes s'étendant tangentiellement de sa paroi périphérique extérieure et étant toutes orientées dans le même sens de rotation, chaque languette étant disposée entre deux fentes adjacentes, chaque languette étant apte à pénétrer à l'intérieur d'une ouverture de la couronne lorsque l'élément nomade est en place sur la partie fixe et que l'utilisateur tourne l'élément nomade. Ainsi, la couronne peut s'accrocher sur la base, et l'utilisateur peut séparer facilement la structure décorative de la couronne, afin de remplacer le corps poreux mobile ;
- les lèvres d'accroche inférieure et supérieure de la couronne sont aptes à se rabattre sur le corps poreux mobile par la déformation radiale élastique de la couronne en fin d'emboitement des languettes dans les ouvertures, pour séparer la couronne de la structure décorative.

L'invention concerne également un flacon comprenant un réservoir apte à stocker un produit fluide, du type parfum, et comprenant un dispositif de diffusion tel que décrit ci-dessus.

### Présentation des figures

L'invention sera mieux comprise, et d'autres buts, détails, caractéristiques et avantages de celle-ci apparaîtront plus clairement au cours de la description explicative détaillée qui va suivre, d'au moins un mode de réalisation de l'invention donné à titre d'exemple purement illustratif et non limitatif, en référence aux dessins schématiques annexés.

Sur ces dessins :
- la figure 1 est une vue éclatée du dispositif de diffusion selon l'invention, avec quelques variantes de pièces proposées à droite ;
- la figure 2 est une vue en perspective d'un élément nomade prêt à être monté sur une partie fixe du dispositif de diffusion selon la figure 1 ;
- la figure 3 montre, en perspective, l'élément nomade monté sur la partie fixe selon la figure 2 ;
- la figure 4 illustre trois parties fixes sur lesquelles peuvent être montés trois éléments nomades ;
- la figure 5 est une vue en coupe et en perspective d'une partie fixe avec sa frette en position haute de protection, montée sur un réservoir ;
- la figure 6 est une vue en coupe et en perspective d'une partie fixe avec sa frette en position basse de libre accès, montée sur un réservoir ;
- la figure 7 est une vue en coupe de l'élément nomade monté sur la partie fixe ;
- la figure 8 est une vue en coupe de l'élément nomade à distance de la partie fixe ;
- la figure 9 représente une première configuration possible d'une couronne appartenant à l'élément nomade ;
- la figure 10 représente une seconde configuration possible d'une couronne appartenant à l'élément nomade ;
- la figure 11 est une vue en perspective est partiellement en coupe d'un élément nomade monté sur une partie fixe avec la couronne de l'élément nomade fixée dans la base de la partie fixe ;
- la figure 12 est une vue en coupe transversale du dispositif de diffusion selon la figure 7 avec la couronne de l'élément nomade désolidarisée de la base de la partie fixe ;
- la figure 13 est une vue en coupe longitudinale du dispositif de diffusion selon la figure 7 avec la couronne de l'élément nomade désolidarisée de la base de la partie fixe ;
- la figure 14 et une vue en coupe transversale du dispositif de diffusion selon la figure 11 avec la couronne de l'élément nomade solidarisée à la base de la partie fixe ;
- la figure 15 est une vue en coupe longitudinale du dispositif de diffusion selon la figure 11 avec la couronne de l'élément nomade solidarisée à la base de la partie fixe ;
- la figure 16 est une vue agrandie et en perspective d'une base d'une partie fixe.

### Description détaillée

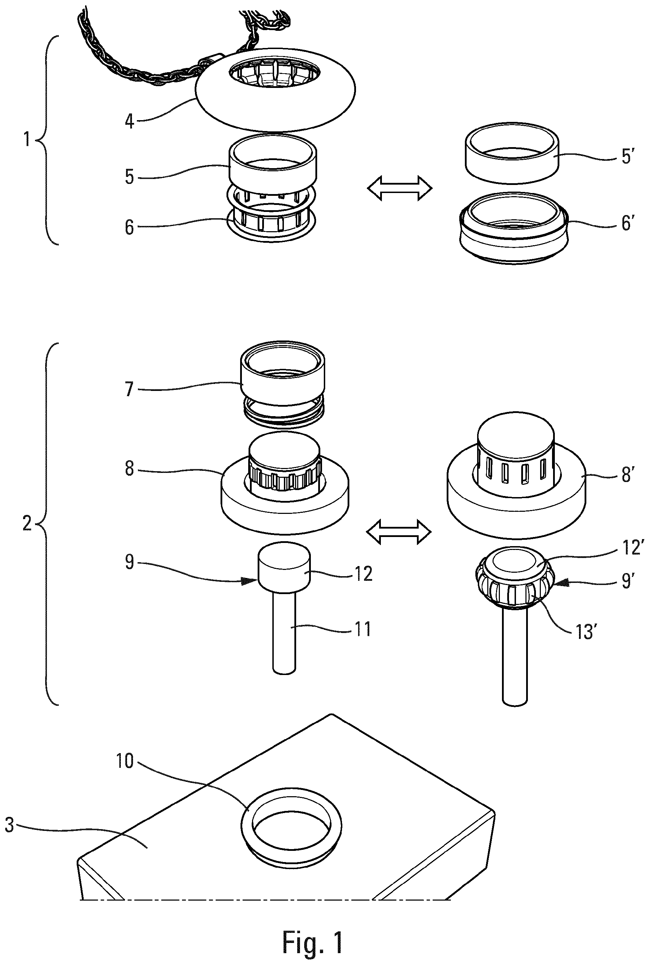

En référence à la figure 1, le dispositif de diffusion selon l'invention se compose principalement d'un élément nomade 1 apte à être monté sur une partie fixe 2, qui elle-même vient se monter sur un réservoir 3.

Ce réservoir 3 contient un produit liquide qui consiste en un produit cosmétique, plus particulièrement du parfum ou une eau de toilette.

Ce réservoir 3 présente un col 10 définissant une ouverture supérieure dans laquelle peut être inséré un corps poreux de la partie fixe 2 du dispositif de diffusion, appelé alors corps poreux fixe 9. Ce corps poreux fixe 9 s'étend sur toute la hauteur du réservoir 3 de manière à ce qu'il puisse toucher le fond du réservoir 3 pour être imbibé de produit, quel que soit le niveau de remplissage de produit dans le réservoir 3.

Ce corps poreux fixe 9 est emmanché dans une base 8 de la partie fixe 2, apte à montée sur le col 10 du réservoir 3. Une frette 7, dont le fonctionnement sera révélé plus tard dans la description, fait également partie de la partie fixe 2.

L'élément nomade 1 quant à lui consiste principalement en un bijou 4 portatif qu'un utilisateur peut porter toute la journée, du type pendentif, qui diffuse le même parfum que celui contenu dans le réservoir 3. Pour cela, l'élément nomade 1 comprend également un corps poreux, appelé corps poreux mobile 5. Ce corps poreux mobile 5 peut s'imbiber de parfum lorsqu'il est mis en contact avec corps poreux fixe 9, lors de périodes de recharge en parfum. Ce corps poreux mobile 5 diffuse le parfum tout au long de la journée par évaporation.

Une couronne 6 fait également partie de l'élément nomade 1, afin de supporter le corps poreux mobile 5, et de maintenir ce dernier à distance du bijou 4, afin que le parfum présent sur le corps poreux mobile 5 n'entre pas en contact avec la structure du bijou 4 de manière à ne pas l'altérer, notamment en termes de corrosion par exemple.

Sur cette figure 1, certaines pièces de l'élément nomade 1 et certaines pièces de la partie fixe 2 peuvent proposer des variantes de forme, leurs fonctions restant identique. Ces variantes représentées sur la droite sont numérotées avec des « primes » pour plus de clarté.

Dans le mode de réalisation présenté ici, l'élément nomade 1 est donc principalement constitué de trois pièces de formes annulaires, à savoir une structure décorative 4 du type bijou, un corps poreux 5 mobile, et une couronne 6. Le bijou 4 est par exemple de forme torique.

Dans la première variante, le corps poreux mobile 5 est monté en force tout autour de la couronne 6 puis l'ensemble couronne 6 / corps poreux mobile 5 est monté à l'intérieur du bijou 4. La couronne 6 assure alors une fonction esthétique, car elle permet de masquer le corps poreux mobile 5 lorsque l'utilisateur regarde au centre du bijou 4.

Dans la seconde variante, le corps poreux mobile 5' est monté à l'intérieur de la couronne 6', puis l'ensemble couronne 6' / corps poreux mobile 5' est monté à l'intérieur du bijou 4.

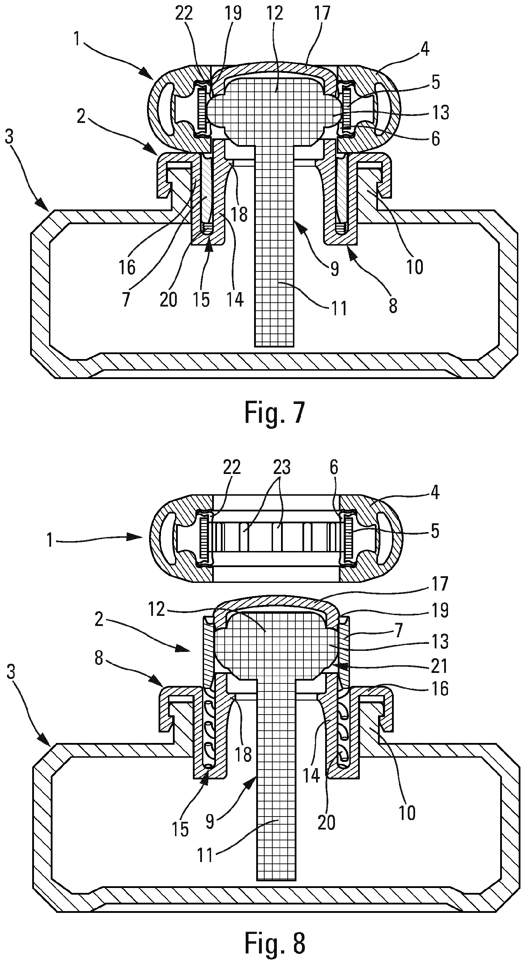

Dans les deux cas, la couronne 6, 6' assure sa fonction de support du corps poreux mobile 5, 5', et sa fonction de mise en retrait du corps poreux mobile 5, 5' par rapport au bijou 4 pour éviter tout contact entre eux. Pour cela, comme illustré aux figures 7, 8 et 9, la couronne 6 comporte une lèvre d'accroche supérieure 26 et une lèvre d'accroche inférieure 27 pour sa fixation avec le bijou 4.

Dans la première variante, le corps poreux mobile 5 est plaqué contre la paroi périphérique de la couronne 6, et se retrouve positionné entre ces deux lèvres 26, 27. Ces deux lèvres 26, 27 présentent une extension radiale supérieure à l'épaisseur du corps poreux mobile 5 de manière à ce qu'elles le recouvrent entièrement et que ce dernier ne puisse pas toucher le bijou 4. Cette extension radiale s'apparente à un porte-à-faux dimensionné de manière à conserver une certaine distance entre le corps poreux mobile 5 et le bijou 4. Le bijou 4 en lui-même comporte une partie creuse annulaire dans laquelle vient s'insérer la couronne 6. Cette partie creuse est dimensionnée de manière à former un logement suffisamment grand pour éviter tout contact avec le corps poreux mobile 5.

Dans la seconde variante, le corps poreux mobile 5' est à l'intérieur de la couronne 6', donc cette dernière fait office de barrière entre le corps poreux mobile 5' et le bijou 4.

Dans les deux cas, le corps poreux mobile 5 doit être accessible depuis l'intérieur du bijou 4, pour sa recharge en parfum. Ainsi dans la première variante, la couronne 6 possède des ouvertures 23 réparties régulièrement sur toute sa périphérie. Des portions du corps poreux mobile 5 sont donc accessibles à travers les ouvertures 23. L'épaisseur du corps poreux mobile 5 est dimensionnée de manière à ce que ses portions disposées en vis-à-vis des ouvertures 23 ne soient pas en saillie vers l'intérieur de l'élément nomade 1. Ainsi, lors de la prise en main de l'élément nomade 1, il n'existe pas de risque de contact entre la peau du doigt d'un utilisateur et lesdites portions de l'élément poreux mobile 5, ou encore entre un vêtement et lesdites portions de l'élément poreux mobile 5. Dans la seconde variante, puisque le corps poreux mobile 5' est monté à l'intérieur de la couronne 6', il est directement accessible en intégralité.

Dans la suite de la description, la première variante sera reprise par les figures. Il est à noter que la seconde variante aurait pu être choisie, de la même manière.

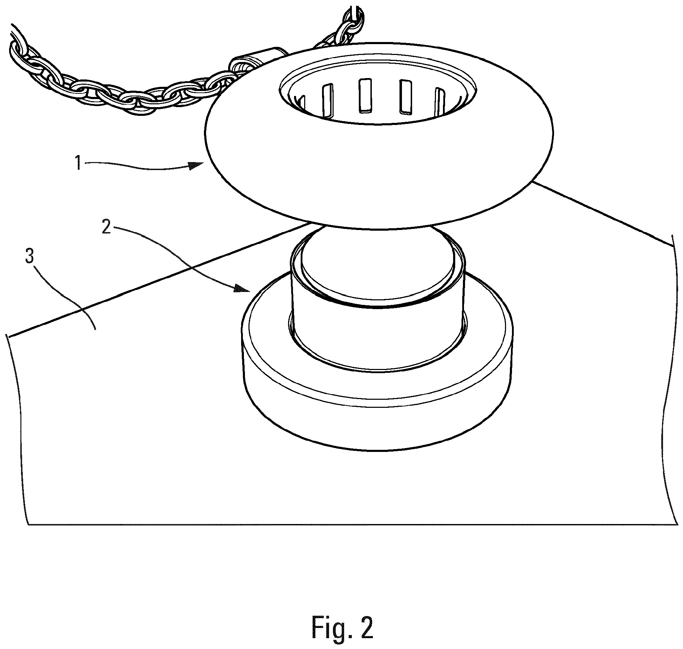

En figure 2, l'élément nomade 1 est entièrement assemblé. De la même manière, la partie fixe 2 est entièrement assemblée et fixée sur le col 10 du réservoir 3. Cette partie fixe 2 présente un seul point d'alimentation. L'élément nomade 1 se trouve à distance de ce point d'alimentation et est prêt à y être rechargé.

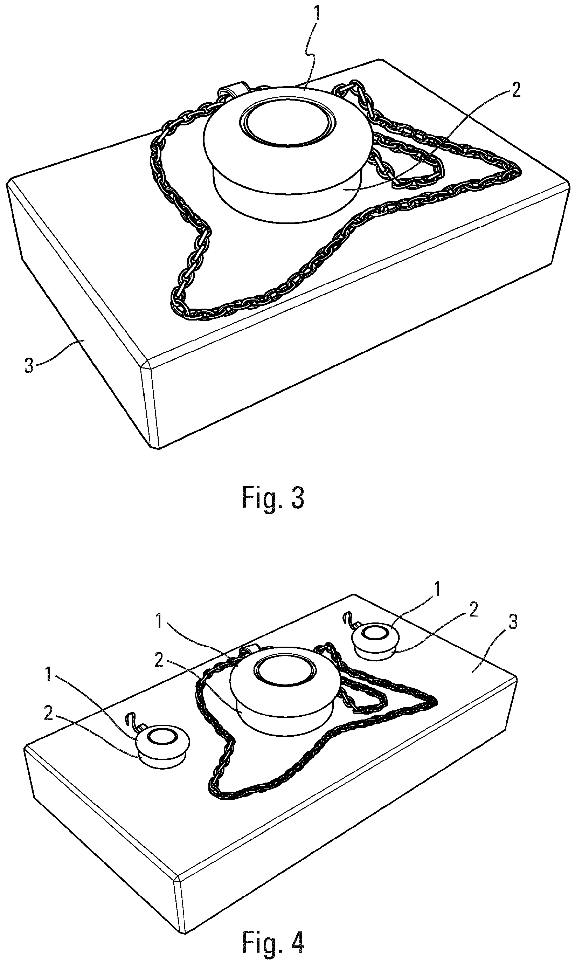

En figure 3, l'élément nomade 1 est monté sur l'unique point d'alimentation de la partie fixe 2 et est en cours de recharge en parfum. Sur cette figure 3, il y a une seule partie fixe 2, présentant un unique point d'alimentation, pour recharger un seul élément nomade 1.

Alternativement, comme illustré en figure 4, il est possible de mettre en place trois parties fixes 2 sur un seul réservoir 3, présentant chacune un seul point d'alimentation, de manière à pouvoir recharger trois éléments nomades 1 simultanément. Dans l'exemple illustré, il y a un pendentif et deux boucles d'oreilles qui peuvent être rechargés simultanément, faisant partie d'une même parure de bijoux 4.

Alternativement, il est également possible de prévoir une partie fixe 2 présentant plusieurs points d'alimentation, afin de pouvoir recharger plusieurs éléments nomades 1 simultanément à partir d'une seule partie fixe 2. Dans ce cas, soit il y a un seul corps poreux fixe 9 inséré à travers un seul col 10, et qui se divise en plusieurs branches offrant alors plusieurs points d'alimentation, soit il y a plusieurs corps poreux fixes 9 saillants du réservoir 3 offrant alors plusieurs points d'alimentation.

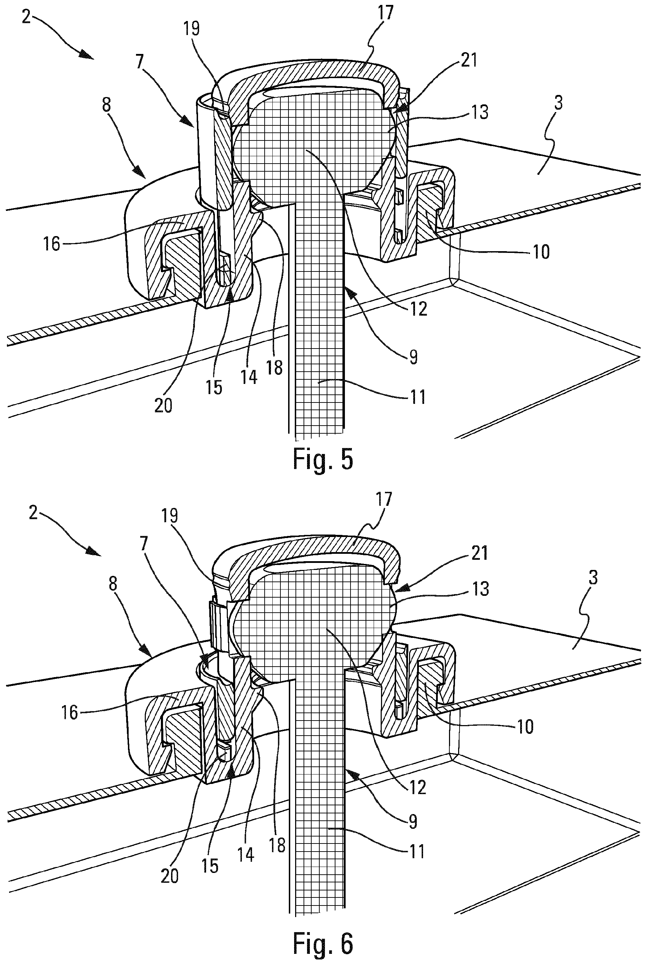

De façon plus précise, comme illustré en figure 6, la partie fixe 2 du dispositif de diffusion comprend une base 8 de fixation sur le réservoir 3. Cette base 8 comporte un manchon 14 central fermé en partie supérieure par un chapeau 17 et ouvert en partie inférieure. Ce manchon 14 définit un logement interne pour le corps poreux fixe 9. En effet, le corps poreux fixe 9 est introduit en force à l'intérieur de ce manchon 14 et se trouve en état de compression. De façon plus précise, le corps poreux fixe 9 comporte une tête 12 s'insérant à l'intérieur du logement tel que défini ci-dessus, et de laquelle s'étend une tige 11 présentant une longueur suffisante pour atteindre le fond du réservoir 3. Le corps poreux fixe 9 est maintenu en position dans le logement grâce à la compression de sa tête 12 dans le logement, et grâce à un rebord annulaire 18 pratiqué sur la surface interne du manchon 14, permettant de retenir la tête 12 si elle venait à descendre.

La paroi périphérique du manchon 14 est ajourée en partie supérieure par des fentes 21 à travers lesquelles saillent des excroissances 13 décomprimées du corps poreux fixe 9. En effet puisque ce dernier est comprimé, il a tendance à s'engouffrer à l'intérieur des fentes 21 afin de se détendre.

Dans la première variante illustrée en figure 1, la tête 12 du corps poreux fixe 9 présente une surface régulière avant son insertion dans le manchon 14, puis cette tête 12 est déformée par compression après insertion, donnant lieu à ces excroissances 13 venant s'engouffrer dans les fentes 21.

Dans la seconde variante illustrée en figure 1, la tête 12' du corps poreux fixe 9' présente une surface irrégulière avant son insertion dans le manchon 14. En effet, elle comporte déjà des excroissances 13' taillées à l'avance dans la masse poreuse. Ces excroissances 13' sont réparties de manière régulière autour de la tête 12', en respectant les dimensions et les distances des fentes 21 prévues à cet effet dans le manchon 14 de la base 8', de manière à ce que chaque excroissance 13' puisse pénétrer à l'intérieur d'une fente 21 correspondante. Ces excroissances 13' sont forcément décomprimées puisqu'aucune force n'est appliquée sur elles. Cette seconde variante n'empêche pas la compression du corps poreux fixe 9' à l'intérieur du manchon 14.

En revenant à la figure 6, la base 8 comporte une jupe 16 se développant autour de la partie inférieure du manchon 14. Cette jupe 16 comprend des moyens de fixation au col 10 du réservoir 3. En l'espèce, la jupe 16 présente un retour annulaire apte à coopérer avec un épaulement annulaire prévu sur le col 10 du réservoir 3, de manière à assurer un emboîtement élastique entre la jupe 16 et le col 10 du réservoir 3.

Lorsque cette partie fixe 2 est ainsi montée sur le col 10 du réservoir 3, le corps poreux fixe 9 s'imbibe automatiquement du parfum présent à l'intérieur du réservoir 3. Le parfum rentre tout d'abord dans la tige 11 puis remonte par capillarité jusqu'à la tête 12 du corps poreux fixe 9. Ainsi, les excroissances 13 du corps poreux fixe 9 sortant des fentes 21 de la base 8 sont toutes imbibées de parfum. Il suffit alors de mettre en place l'élément nomade 1 sur la partie supérieure du manchon 14, de manière à ce que l'élément nomade 1 entoure toutes les fentes 21 du manchon 14, comme cela est visible en figure 7. Ainsi, les excroissances 13 du corps poreux fixe 9, imbibées de parfum, viennent au contact du corps poreux mobile 5 qui est devenu sec après une journée de diffusion, ou au moins au contact des portions du corps poreux mobile 5 accessibles via les ouvertures 23 de la couronne. Par capillarité, le parfum passe du corps poreux fixe 9 au corps poreux mobile 5.

De préférence, l'utilisateur met en place son bijou 4 sur la base 8 le soir au coucher, de manière à ce que la recharge se produise pendant la nuit, et que le bijou 4 soit à nouveau prêt à emploi et imbibé de parfum le lendemain matin.

Lorsqu'il n'y a pas d'élément nomade 1 positionné sur la base 8, les fentes 21 et les excroissances 13 du corps poreux fixe 9 sont à l'air libre. Par conséquent, le parfum est diffusé en permanence dans l'air ambiant à partir de la partie fixe 2. Pour éviter cette diffusion, et que le réservoir 3 ne se vide pas de cette manière, il est nécessaire de prévoir des moyens de protection contre l'évaporation du produit.

Comme illustré aux figures 5 et 6, ces moyens de protection contre l'évaporation du produit consistent en une frette 7 mobile en translation à l'encontre de moyens ressorts entre une position haute de protection en vis-à-vis des fentes 21 de la base 8 et une position basse de libre accès aux fentes 21 pour l'élément nomade 1.

Sur la figure 5, la frette 7 est en position haute et recouvre intégralement toute la partie supérieure périphérique du manchon 14, de manière à former une barrière contre l'évaporation du parfum à travers les fentes 21. Mieux encore, le chapeau 17 du manchon 14 comporte un rebord périphérique 19 contre lequel la frette 7 vient en butée en position haute, pour assurer l'étanchéité totale entre la base 8 et la frette 7. Ainsi, lorsqu'il n'y a pas d'élément nomade 1 en place sur la partie fixe 2, la base 8 est totalement étanche et le parfum ne peut pas se diffuser.

Plus précisément, les moyens ressorts de la frette 7 consistent en des pattes 20 flexibles reposant au fond d'une gorge 15 formée entre le manchon 14 et la jupe 16 de la base 8. Ces pattes 20 flexibles peuvent prendre la forme d'un ressort torsadé, ou peuvent simplement correspondre à une pluralité de pattes 20 aptes à fléchir sous l'effet d'une contrainte. Toute forme de patte peut être envisagée.

Sur la figure 6, la frette 7 est en position basse de libre accès aux fentes 21 pour l'élément nomade 1. En effet, sous l'effet d'une contrainte exercée sur la frette 7, les pattes 20 fléchissent et la frette 7 translate en direction du fond de la gorge 15 jusqu'à se cacher totalement à l'intérieur de la gorge 15. Les fentes 21 avec les excroissances 13 du corps poreux fixe 9 sont ainsi à découvert, et diffusent du parfum.

Sur la figure 8, la frette 7 est en position haute puisque l'élément nomade 1 se trouve à distance de la partie fixe 2. Aucune diffusion de parfum n'est donc réalisée au niveau de la partie fixe 2.

Sur la figure 7, l'utilisateur vient mettre en place l'élément nomade 1 sur la base 8 de la partie fixe 2. Lors de la mise en place, le bijou 4 va exercer une force sur la frette 7 et va l'entraîner en translation vers sa position basse. L'élément nomade 1 se retrouve alors en position tout autour de la partie supérieure du manchon 14 de la base 8, et le corps poreux fixe 9 est en contact avec le corps poreux mobile 5, le transfert de parfum par capillarité ayant alors lieu, comme nous l'avons vu précédemment.

L'élément nomade 1 peut rester en place autour du manchon 14 car il est dimensionné de telle manière qu'il exerce un frottement radial et axial contre le manchon 14, qui est suffisant pour résister à l'encontre de la force de rappel des pattes 20 flexibles de la frette 7. En effet, le diamètre intérieur du bijou 4 est quasiment identique au diamètre extérieur du manchon 14 de manière à ce que la mise en place de l'élément nomade 1 sur le manchon 14 se fasse légèrement en force. De plus, la couronne 6 de l'élément nomade 1 comporte une légère forme bombée au niveau de sa jonction entre sa paroi périphérique et ses lèvres supérieures et inférieures, qui vient se positionner sous le rebord périphérique 19 du chapeau 17 du manchon 14, permettant ainsi de mieux maintenir en position l'élément nomade 1 sur la base 8.

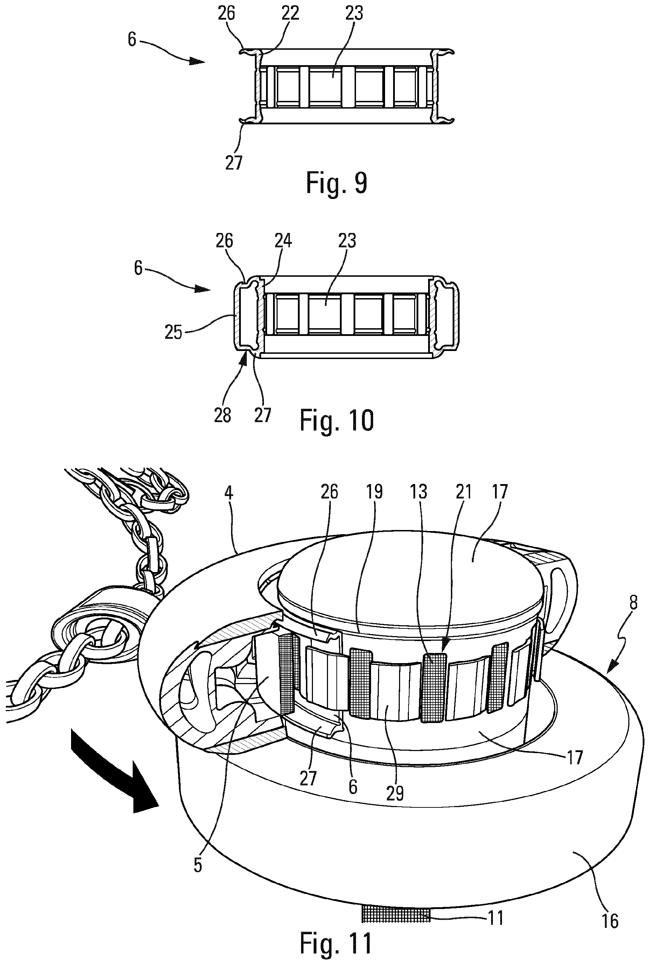

La figure 9 montre une première configuration possible d'une couronne 6 appartenant à l'élément nomade 1. Comme vu précédemment, cette couronne 6 comporte des ouvertures 23 ainsi qu'une lèvre supérieure 26 et une lèvre inférieure 27. Les lèvres 26,27 ont une extension radiale suffisante pour couvrir l'épaisseur du corps poreux mobile 5.

Il est également envisageable de prolonger ces lèvres 26,27 par un bouclier 25 de protection venant alors entourer complètement le corps poreux mobile 5, comme illustré en figure 10, pour former une cage de protection. Dans ce cas, un interstice 28 est pratiqué entre le bouclier 25 et la lèvre inférieure 27 afin de permettre l'insertion du corps poreux mobile 5 dans le volume défini entre le bouclier 25 et la paroi périphérique de la couronne 6. Dans ce cas, le corps poreux mobile 5 est totalement caché derrière le bouclier 25. Le bijou 4 est totalement protégé par rapport au corps poreux mobile 5.

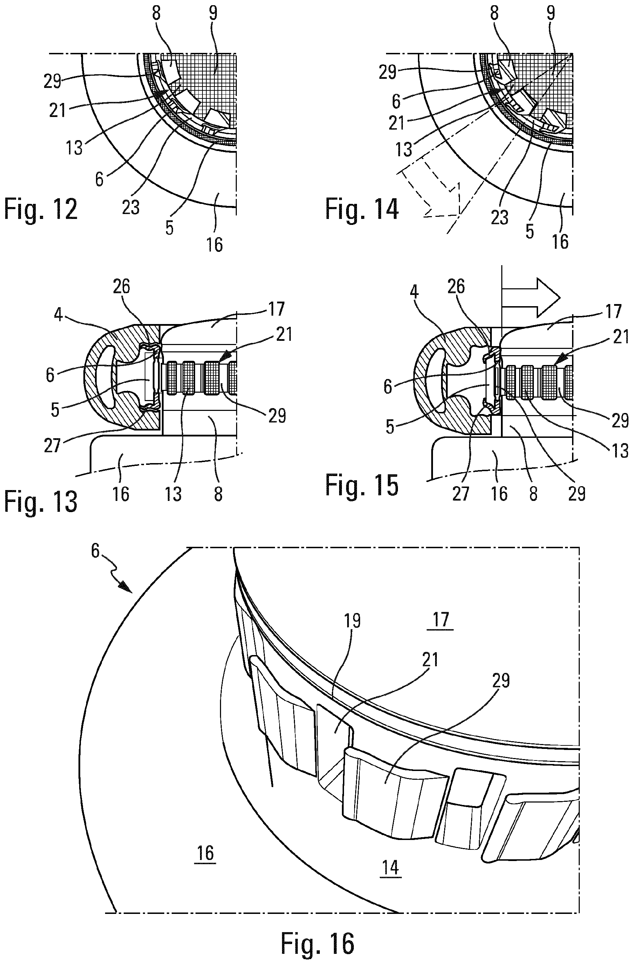

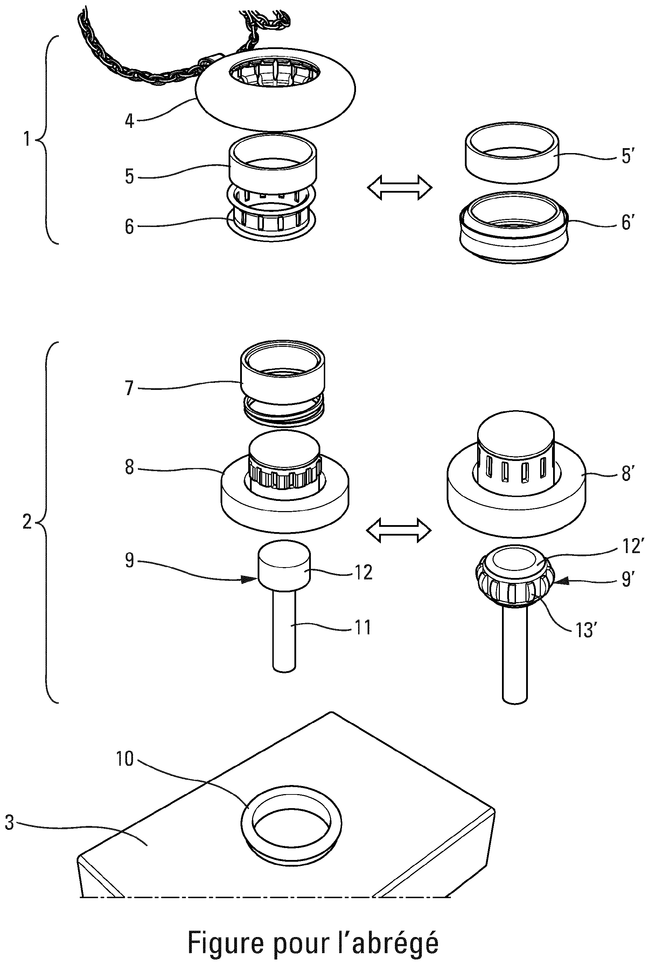

Lors du remplacement du corps poreux mobile 5, la couronne 6 de l'élément nomade 1 s'accroche au manchon 14 de la partie fixe 2 afin de pouvoir être désolidarisée du bijou 4, et que le remplacement du corps poreux mobile 5 puisse être fait. Pour cela, des languettes 29 d'accroche sont prévues sur le manchon 14 de la base 8. Ces languettes 29 sont notamment illustrées sur les figures 11 et 16. Plus précisément, ces languettes 29 s'étendent tangentiellement de la paroi périphérique externe du manchon 14, et sont toutes orientées dans le même sens de rotation. Chaque languette 29 est disposée entre deux fentes 21 adjacentes. Chaque languette 29 est apte à pénétrer à l'intérieur d'une ouverture 23 de la couronne 6 lorsque l'élément nomade 1 est en place sur la partie fixe 2 et que l'utilisateur tourne l'élément nomade 1.

Sur les figures 12 et 13, l'élément nomade 1 est simplement posé et enfoncé sur le manchon 14, pour une recharge classique de parfum par capillarité.

Si l'utilisateur souhaite remplacer l'élément poreux mobile, par exemple parce qu'il est trop vieux, il lui suffit de tourner l'élément nomade 1 par rapport à la base 8 comme indiqué par les flèches aux figures 14 et 15. Grâce à cette rotation, les languettes 29 pénètrent à l'intérieur des ouvertures 23 de la couronne 6, et cette dernière se retrouve ainsi solidarisée au manchon 14. L'utilisateur peut alors retirer le bijou 4 seul par rapport à la base 8. La couronne 6 et le corps poreux mobile 5 restent quant à eux accrochés à la base 8. L'utilisateur peut alors facilement enlever le corps poreux mobile 5 de la couronne 6 et en mettre un autre. Pour solidariser à nouveau le bijou 4 à la couronne 6, il suffit de faire l'opération inverse, c'est-à-dire d'insérer à nouveau le bijou 4 sur le manchon 14 via un mouvement de translation axiale, puis de tourner le bijou 4 par rapport à la base 8 dans le sens inverse de manière à ce que les languettes 29 se retirent des ouvertures 23 de la couronne 6, et que la couronne 6 se retrouve ainsi à nouveau en prise à l'intérieur du bijou 4. L'élément nomade 1 peut alors être retiré à nouveaux en entier par rapport à la base 8.

Il en va de même lors de la première utilisation du corps poreux mobile 5. En effet, à la première utilisation, la couronne 6 est solidarisée au manchon 14 comme illustré aux figures 14 et 15, avec le corps poreux mobile 5. L'utilisateur peut alors choisir le bijou 4 qu'il préfère, puis vient le positionner et l'enfoncer sur le manchon 14. Il lui suffit alors de tourner le bijou 4 dans le sens inverse des flèches de manière à retirer les languettes 29 des ouvertures 23 de la couronne 6. La couronne 6 et le corps mobile poreux 5 se retrouvent alors emboités à l'intérieur du bijou 4, et l'utilisateur peut retirer l'intégralité de l'élément nomade 1 par rapport à la base 8.

Lorsque les languettes 29 sont entièrement emboîtées dans les ouvertures 23, la couronne 6 a alors tendance à se déformer radialement. En effet, la couronne 6 est fabriquée à partir d'un matériau présentant des propriétés élastiques, lui permettant de se déformer élastiquement. En l'espèce, en continuant son mouvement de rotation, l'utilisateur va déformer la paroi périphérique de la couronne 6 radialement vers l'intérieur, vu l'accroche en place avec les languettes 29. Cette déformation radiale va engendrer une déformation angulaire des lèvres supérieure 26 et inférieure 27, qui vont avoir tendance à se rapprocher l'une de l'autre pour venir se refermer sur le corps poreux mobile 5.

Optionnellement, les lèvres supérieure 26 et inférieure 27 de la couronne 6 présentent des zones de faiblesse au niveau de leur jonction avec la paroi périphérique de la couronne 6. Ces zones de faiblesse permettent de mieux fléchir les lèvres 26,27 de manière à les rabattre encore plus près du corps poreux mobile 5, comme cela est illustré en figure 15.

Lorsque les deux lèvres 26,27 sont rabattues, il est bien plus aisé pour l'utilisateur de retirer le bijou 4 par rapport à la couronne 6, cette dernière étant solidarisée au manchon 14 via les languettes 29.

Les configurations montrées aux figures citées ne sont que des exemples possibles, nullement limitatifs, de l'invention qui englobe au contraire les variantes de formes et de conceptions à la portée de l'homme de l'art.

## Revendications

1. Dispositif de diffusion d'un produit liquide, du type parfum, stocké dans un réservoir 3, comprenant :
- au moins une partie fixe 2 apte à être montée sur le réservoir 3, et offrant au moins un point d'alimentation en produit par capillarité, ladite partie fixe 2 comprenant un corps poreux fixe 9 apte à s'imbiber de produit par capillarité en étant directement plongé dans le réservoir 3 ;
- un élément nomade 1, pour chaque point d'alimentation, comprenant un corps poreux mobile 5 apte à s'imbiber de produit par capillarité en étant en contact avec le corps poreux fixe 9 lorsque l'élément nomade 1 est monté sur la partie fixe 2, ledit élément nomade 1 comprenant également une structure décorative 4, ledit corps poreux mobile 5 étant logé à l'intérieur de la structure décorative 4.

2. Dispositif de diffusion selon la revendication précédente, **caractérisé en ce que** l'élément nomade 1 comprend des moyens de mise en retrait du corps poreux mobile 5 par rapport à la structure décorative 4 pour éviter tout contact direct entre eux.

3. Dispositif de diffusion selon l'une des revendications précédentes, **caractérisé en ce que** la partie fixe 2 comprend une base 8 adaptée à être fixée sur le réservoir 3 et retenant le corps poreux fixe 9 en état de compression, ladite base 8 étant ajourée par des fentes 21 à travers lesquelles saillent des excroissances 13 décomprimées du corps poreux fixe 9.

4. Dispositif de diffusion selon la revendication précédente, **caractérisé en ce que** ladite base 8 comporte un manchon 14 fermé en partie supérieure par un chapeau 17 et ouvert en partie inférieure de manière à définir un logement pour le corps poreux fixe 9, lesdites fentes 21 étant pratiquées sur la paroi périphérique du manchon 14.

5. Dispositif de diffusion selon la revendication précédente, **caractérisé en ce que** ladite base 8 comporte une jupe 16 se développant autour de la partie inférieure du manchon 14, ladite jupe 16 comprenant des moyens de fixation adaptés au col 10 du réservoir 3.

6. Dispositif de diffusion selon la revendication précédente, **caractérisé en ce que** la partie fixe 2 comporte des moyens de protection contre l'évaporation du produit à travers lesdites fentes 21.

7. Dispositif de diffusion selon la revendication précédente, **caractérisé en ce que** lesdits moyens de protection contre l'évaporation du produit consistent en une frette 7 mobile en translation à l'encontre de moyens ressort entre une position haute de protection en vis-à-vis des fentes 21 et une position basse de libre accès aux fentes 21 pour l'élément nomade 1.

8. Dispositif de diffusion selon la revendication précédente, **caractérisé en ce que** lesdits moyens ressort de ladite frette 7 consistent en des pattes 20 flexibles reposant au fond d'une gorge 15 formée entre le manchon 14 et la jupe 16 de la base 8.

9. Dispositif de diffusion selon la revendication précédente, **caractérisé en ce que** le chapeau 17 comporte un rebord périphérique 19 contre lequel la frette 7 vient en butée en position haute pour assurer l'étanchéité entre la base 8 et la frette 7 lorsque l'élément nomade 1 n'est pas en place sur la partie fixe 2.

10. Dispositif de diffusion selon l'une des revendications 7 à 9, **caractérisé en ce que** le corps poreux mobile 5 consiste en un élément annulaire entourant la paroi périphérique ajourée du manchon 14 lorsque l'élément nomade 1 est en place sur la partie fixe 2, l'élément nomade 1 exerçant une force sur la frette 7 de manière à la maintenir en position basse par un effet de frottement.

11. Dispositif de diffusion selon l'une des revendications 2 à 10, **caractérisé en ce que** lesdits moyens de mise en retrait du corps poreux mobile 5 par rapport à la structure décorative 4 de l'élément nomade 1 consistent en une couronne 6 supportant le corps poreux mobile 5, ladite couronne 6 étant fixée de manière amovible dans la structure décorative 4.

12. Dispositif de diffusion selon la revendication précédente, lorsqu'elle dépend de la revendication 3, **caractérisé en ce que** le corps poreux mobile 5 est disposé à l'intérieur de la couronne 6, ledit corps poreux mobile 5 venant directement au contact des excroissances 13 décomprimées du corps poreux fixe 9 lorsque l'élément nomade 1 est en place sur la partie fixe 2.

13. Dispositif de diffusion selon la revendication 11 lorsqu'elle dépend de la revendication 3, **caractérisé en ce que** le corps poreux mobile 5 est disposé à l'extérieur de la couronne 6, la couronne 6 étant ajourée par des ouvertures 23 à travers lesquelles des portions du corps poreux mobile 5 sont accessibles, lesdites excroissances 13 décomprimées du corps poreux fixe 9 venant au contact de ces portions du corps poreux mobile 5 lorsque l'élément nomade 1 est en place sur la partie fixe 2.

14. Dispositif de diffusion selon la revendication précédente, **caractérisé en ce que** la couronne 6 comporte une lèvre d'accroche supérieure 26 et une lèvre d'accroche inférieure 27 pour sa fixation avec la structure décorative 4, lesdites lèvres 26,27 ayant une extension radiale dimensionnée de manière à assurer une mise en retrait du corps poreux mobile 5 par rapport à la structure décorative 4.

15. Dispositif de diffusion selon l'une des revendications 13 à 14, **caractérisé en ce que** le manchon 14 comporte des languettes 29 s'étendant tangentiellement de sa paroi périphérique extérieure et étant toutes orientées dans le même sens de rotation, chaque languette 29 étant disposée entre deux fentes 21 adjacentes, chaque languette 29 étant apte à pénétrer à l'intérieur d'une ouverture 23 de la couronne 6 lorsque l'élément nomade 1 est en place sur la partie fixe 2 et que l'utilisateur tourne l'élément nomade 1.

16. Dispositif de diffusion selon la revendication précédente, **caractérisé en ce que** les lèvres d'accroche inférieure 27 et supérieure 26 de la couronne 6 sont aptes à se rabattre sur le corps poreux mobile 5 par la déformation radiale élastique de la couronne en fin d'emboitement des languettes 29 dans les ouvertures 23, pour séparer la couronne 6 de la structure décorative 4.

17. Flacon comprenant un réservoir 3 apte à stocker un produit fluide, du type parfum, et comprenant un dispositif de diffusion selon l'une des revendications précédentes.
